(19) Europäisches Patentamt / European Patent Office / Office européen des brevets

(11) **EP 2 506 764 B1**

(12) **EUROPEAN PATENT SPECIFICATION**

(45) Date of publication and mention of the grant of the patent:
**15.03.2017 Bulletin 2017/11**

(21) Application number: **11706652.2**

(22) Date of filing: **06.01.2011**

(51) Int Cl.:
*A61B 5/053* (2006.01)

(86) International application number:
**PCT/US2011/020359**

(87) International publication number:
**WO 2011/085090 (14.07.2011 Gazette 2011/28)**

(54) **METHOD OF MANAGING A WEIGHT CONDITION IN AN ANIMAL**

VERFAHREN ZUR VERWALTUNG VON GEWICHTSSTÖRUNGEN BEI TIEREN

PROCÉDÉ DE GESTION D'UN ÉTAT PONDÉRAL CHEZ UN ANIMAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.01.2010 US 292652 P**

(43) Date of publication of application:
**10.10.2012 Bulletin 2012/41**

(73) Proprietor: **Hill's Pet Nutrition, Inc.**
**Topeka, KS 66603 (US)**

(72) Inventors:
• **TOLL, Philip W.**
**Valley Falls**
**Kansas 66088 (US)**
• **PAETAU-ROBINSON, Inke**
**Auburn**
**Kansas 66402 (US)**
• **KIRK, Claudia A.**
**Louisville**
**Tennessee 37777-3424 (US)**

(74) Representative: **Daniels, Jeffrey Nicholas**
**Page White & Farrer**
**Bedford House**
**John Street**
**London WC1N 2BF (GB)**

(56) References cited:
**WO-A1-2005/089567     WO-A2-96/19141**
**US-A1- 2004 068 379**

• **DATABASE COMPENDEX [Online] ENGINEERING INFORMATION, INC., NEW YORK, NY, US; December 2006 (2006-12), BARTHELMESS E L ET AL: "The value of bioelectrical impedance analysis vs. condition indices in predicting body fat stores in North American porcupines (Erethizon dorsatum)", XP002638562, Database accession no. E20071610560614 & CANADIAN JOURNAL OF ZOOLOGY DECEMBER 2006 NATIONAL RESEARCH COUNCIL OF CANADA; PUBLICATION RESEARCH PRESS CA, vol. 84, no. 12, December 2006 (2006-12), pages 1712-1720, DOI: DOI:10.1139/Z06-165**

EP 2 506 764 B1

## Description

### BACKGROUND

The embodiments described herein relate to a methodology of assessing body fat and determining an appropriate weight loss regimen for companion animals. More specifically, described herein is a clinically useful tool and methodology to apply to overweight and obese animals.

Obesity is on the rise in the United States, and not only in humans. In 2008, a companion animal obesity study by the Association for Companion Animal Obesity Prevention concluded that an estimated 84 million U.S. dogs and cats are overweight or obese, accounting for approximately 50% of dogs and cats. Moreover, an estimated 10% of dogs and an estimated 18% of cats are obese. In fact, obesity is considered one of the most common forms of malnutrition occurring in dogs.

Generally, companion animals such as canines and felines weighing more than 15% of their ideal body weight are considered overweight or obese. Overweight animals generally have an excess of body adipose tissue. The most common cause of an animal being overweight is an over consumption of food that results in an excess intake of calories. Studies have shown that fat animals are significantly more at risk for diseases such as arthritis, heart disease, respiratory disease, diabetes, bladder cancer, hypothyroidism, and pancreatitis.

As companion animals become more and more obese, the difficulties presented to the veterinarian or animal practitioner become increasingly apparent. One difficulty realized by many veterinarians is the need to accurately prescribe the amount of food that the companion animal owner should feed to the companion animal in order to attain the optimum level of health for the companion animal. In order to accurately prescribe the amount of food that the companion animal owner should feed the companion animal, the veterinarian must first accurately assess the energy needs of the animal. Likewise, in order to accurately prescribe the energy needs of the animal, the veterinarian must accurately determine the percentage body fat of the animal.

Thus, the process of prescribing the proper amount of food for an appropriate weight loss regimen is ultimately dependent upon, among other things, the accurate calculation of body fat percentage. The more error in the calculation of body fat percentage, the more incorrect the caloric assessment will be.

Currently, the technique of body condition scoring (BCS) is the most accessible and popular method for estimating obesity in companion animals. This method is accessible and popular because of its simplistic use of physical criteria that are easily measurable by the veterinarian or animal practitioner. Under the BCS method, physical examination, visual observation, and palpation may be used to assign a body condition score. The body condition score is a semi-quantitative assessment of body fat with a range of categories from lean to severely obese. The estimates of the BCS method, although inexact, have been confirmed to roughly correlate to the actual body fat percentage as determined by dual-energy X-ray absorptiometry (DEXA).

However, the BCS method is largely ineffective in many instances. Because the BCS method applies the same testing criteria, it attempts a one-size-fits-all solution to a challenging dynamic problem. Additionally, the specific physical parameters that should be measured in order to clinically assess a companion animal's body fat percentage may not be equivalent in each situation. Although anthropomorphic measurements such as skinfold measurements have historically been applied to estimate body fat percentage in humans, these types of measurements have been found to be less effective in companion animals. In essence, the diagnostic procedures for assessing body fat that are currently available to practicing veterinarians and animal practitioners do not remedy the problems associated with the current flawed techniques. For example, while the body fat in humans can be closely estimated using skinfold calipers, the canine triceps is not as cooperative.

While rudimentary methods such as the BCS method are more accurate for companion animals with a low amount of fat, these multiple body condition scoring methods are insufficient to estimate the body fat over the range of obese companion animals. Because an accurate assessment of body fat in an animal is a prerequisite to establishing ideal weight and calculating an accurate caloric dose for weight loss, the margin of error is compounded in the typical procedures for prescribing a weight loss regimen.

Morphometric measurements have been used in dogs and cats, but little has been published comparing objective body measurements with body fat. In part, this is due to the fact that companion animals deposit and store fat subcutaneously in various locations, including the thoracic, lumbar, and coccygeal areas as well as intra-abdominally. When companion animals are subject to weight gain, the pelvic circumference usually changes the most. While specific measurements of the pelvic circumference have at times been used to estimate body fat percentage, this method is also lacking in accuracy and precision.

Because the current methods for estimating body fat percentage of companion animals are often ineffective, the present invention attempts to advance the tools available to the veterinarian and animal practitioner based on objective criteria and statistical analysis. Accordingly, a methodology of assessing body fat and for determining an appropriate weight loss regimen for companion animals is provided. In accordance with the present invention, a method is additionally provided to assist practitioners with practical diagnostic tools to determine body fat and ideal body weight in companion animals, particularly in overweight and obese companion animals. This information is useful for providing a simple means of calculating the energy needs of an animal and an effective food dose for weight loss therapy.

US2004/068379 discloses a method for measuring a body composition measure of an animal which includes the steps of measuring a first electrical impedance between a front foot pad of the animal and a rear foot pad of the animal; measuring a second electrical impedance between two front foot pads of the animal in parallel with one another and two rear foot pads of the animal in parallel with one another; and utilizing the first electrical impedance, the second electrical impedance, and a regression relationship to determine the body composition measure of the animal.

WO2005/089567 discloses compositions useful for weight management in an animal.

Barthelmess *et al* (Compendex, accession no.E20071610560614, Engineering Information Inc, New York, December 2006) discloses the value of bioelectrical impedence analysis vs. condition indices in predicting body fat stores in North American porcupines (*Erethizon dorsatum).*

## BRIEF SUMMARY OF THE INVENTION

The present invention provides a method according to claim 1. Also disclosed herein, but not claimed is a method of managing a weight condition in a companion animal using tools to estimate the body fat percentage of the companion animal is provided. The method includes using the body fat percentage to provide an effective weight loss regimen for the companion animal. Further, the method involves determining the ideal body weight of the companion animal, the daily feeding amount to reduce the companion animal's weight to a desirable level, and the expected weight loss of the companion animal, provided the daily feeding regimen is followed.

The formula for body fat assessment is determined by regression analysis. Using DEXA results or similar reliable methods to determine the actual percentage body fat or lean body mass, physical data may be measured and descriptive data may be used to correlate the data and develop equations to predict percentage body fat or lean body mass based on the measured and descriptive data.

In an embodiment, the formula for body fat assessment is divided into two separate formulas: one formula for animals with body weight less than or equal to a threshold amount, and a separate formula for animals with body weight greater than a threshold amount.

In still a further embodment of the present invention, the animals are dogs and the threshold amount is 18.144 kg (40 pounds).

A method is provided whereby a practitioner may utilize a spreadsheet, program, or similar tool to enter descriptive and measurement information in order to automatically calculate the percentage body fat, the ideal body weight, the resting energy requirements, the food dose amounts, and any other information relating to the weight loss program for the companion animal.

It is to be understood that both the foregoing general description of the invention and the following detailed description are exemplary, but are not restrictive, of the invention.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

## BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:

Figure 1 illustrates a high level flow chart of a method of assessing body fat and determining an appropriate weight loss regimen;

Figure 2 illustrates exemplary methods for the estimation of body fat percentage in companion animals;

Figure 3 illustrates a high level flow chart of a method of first using a reliable process to determine the body fat of a group of animals, and then measuring physical data in order to apply regression analysis to formulate best-fit equations for the clinically-friendly calculation of body fat percentage and the ultimate prescription of a weight loss regimen;

Figure 4 illustrates exemplary input parameters and output parameters for a target weight and food dose calculator for dogs less than or equal to 18.144 kg (40 lbs.);

Figure 5 illustrates exemplary input parameters and output parameters for a target weight and food dose calculator for dogs greater than 18.144 kg (40 lbs).

## DETAILED DESCRIPTION

An exemplary embodiment is a method of managing a weight condition of a companion animal comprising using methods to determine the actual percentage lean body mass of a companion animal; using measured physical data of the companion animal and descriptive data of the companion animal to apply regression analysis based on the actual percentage of body fat or lean body mass; and deriving one or more equations based on the regression analysis, the one or more equations for predicting the percentage body fat or lean body mass of the companion animal based on measured physical data and descriptive data of the new companion animal. In a preferred embodiment, the method to determine the actual percentage of body fat or lean body mass of a companion animal is dual-energy X-ray absorptiometry (DEXA).

The method also includes where the one or more equations are two separate equations, the first equation to be applied to companion animals with body weight less than a threshold amount, and the second equation to be applied to companion animals with body weight greater than a threshold amount.

The method also includes where the companion animals are dogs, and the threshold amount is 18.144 kg (40 pounds).

In still a further embodiment, the animals are cats.

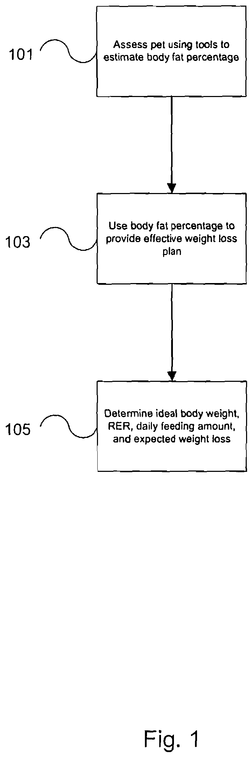

According to **Fig. 1**, the first step in the process is to assess the companion animal using tools to estimate the body fat percentage **101**. As further described below, this can be performed in a variety of fashions. An exemplary methodology described below utilizes body fat assessment tools and a weight loss calculator or similar tool. The companion animal is assessed using criteria to provide a body fat index or score. The body fat index or score may either be based on an estimate of the percentage body fat of the animal, or the actual percentage body fat of the animal. This number is then entered into a calculator or similar tool, which in turn provides the information necessary for an effective weight loss regimen **103**. This information may include the ideal body weight of the animal, the resting energy requirement (RER), the daily feeding amount, and the expected weight loss **105**.

In animal weight assessment, once the body fat percentage has been estimated **101**, the estimated body fat percentage may be used to estimate the RER and the ideal body weight of the animal **105**. Using the current BCS method that is applied to normal-weight animals, the process has the undesirable result of over-estimating the daily caloric need in animals that have excess body fat. As the body fat of the animal increases, the over-estimation of daily caloric need becomes greater and greater. Therefore, the current process further complicates the problem that it was initially designed to address.

The over-estimation resulting from application of the BCS method was recently discovered in an initial study leading to the development of the present invention. More fully described below, the initial study demonstrated that current methods of estimating ideal body weight for weight-loss feeding are largely inaccurate for dogs having greater than 45% body fat.

A further downside to the BCS method is the more obese the animal, the less accurate the method. In fact, the BCS method becomes increasingly inaccurate for animals with body fat percentages above 45%. In part, this is because BCS was designed primarily to assess dogs with body fat percentages at less than 45%. Because of the increasing number of dogs with high-percentages of body fat, BCS as a one-size-fits-all method is becoming less and less effective. For instance, many obese dogs currently have body fat percentage at a level above 50%, for which the BCS method is largely ineffective.

An element of the initial study demonstrated that current methods (i.e., BCS) of estimating ideal body weight for weight loss feeding are inaccurate in dogs having more than 45% body fat. The two major limitations of the current methods of assessing body fat is that (1) precision and accuracy are highly dependent on the training and skill of the individual doing the assessment, and (2) the current body condition scoring scales do not differentiate between different levels of obesity. For example, in the BCS 5 point scale, all dogs with greater than 35% body fat fall into a single score of 5. This has the undesirable effect that a dog with 60% body fat and a dog with 36% body fat both receive the same score. The fatter the dog, the more overestimation of ideal body weight and feeding amount, and therefore the slower and more ineffective the weight loss program.

The initial study compared the accuracy of using body fat percentages to the 5 and 9 point BCS systems for estimating ideal body weight and RER in the dogs. Once a BCS value was assigned by an animal practitioner, the median body fat percentage for each score was used to estimate ideal body weight and RER. Based on the results of the DEXA scans, the body fat ranged from 28.3% to 63.7%, with a mean of 45.9%. In order to assess the accuracy of BCS for moderately versus morbidly obese dogs, the dogs were divided into two groups. The first group had less than 45% body fat, and the second group had greater than 45% body fat. There were 15 dogs in the first group and 21 dogs in the second group.

Compared to DEXA, estimations of ideal body weight were significantly higher using the 5 (23.0 vs. 19.2 kg) and 9 (21.1 vs. 19.2 kg) point BCS in dogs with body fat greater than 45% ($p < 0.001$) but did not differ in dogs with less than 45% ($p > 0.05$).

The results of the above study therefore demonstrate that current BCS systems provide good estimates of ideal body weight and RER in dogs with less than 45% body fat, but are inadequate for calculating RER and ideal body weight in morbidly obese dogs with body fat greater than 45%.

Adding to the problem of assessing the body fat percentage is the error associated with estimating the RER. For an animal weight loss program to remain effective, the daily caloric intake of the animal should be restricted below the level required to maintain the current body weight. In normal-weight animals, the calculation of daily caloric need may be based on the body weight of the animal. However, applying the same approach to above-weight animals can have negative consequences, including over-estimation of the daily caloric needs of the animal.

In addition, a further study described below suggests that DEXA (or equivalent techniques) may be used in combination with known morphometric measurements and basic biological information to use statistical analysis to formulate a best-fit equation, the best-fit equation being appropriate for determining an effective weight loss regimen for any domestic companion animal.

The following is a summarizing description of how morphometric measurements may be taken. A person having ordinary skill in the art will realize that any similar manner of measuring physical attributes may be properly understood as equivalent, and the following is merely exemplary and non-limiting in nature. For instance, body length may be measured by using a Seca measuring rod to measure from the sternum to seat bone/rectum with the companion animal in a normal standing position and head pointing straight forward. Front height may be measured using the Seca floor height rod for measuring the standing height at the shoulder. Rear height may be measured using a Seca floor height rod for measuring the standing height at the hip. Thoracic circumference may be measured using a tailor's tape to wrap the tape tightly around the rib cage at the heart girth when measuring. The pelvic circumference may be measured using a tailor's tape to wrap tightly around the loin area just in front of the knee.

Next, provided herein is a description of the leg measurements. The hind leg length may be measured using a metal tape measure to measure the length of the hind leg from the central foot pad to the dorsal tip of the calcaneal process. Hind leg calcaneus width may be measured using a digital caliper to measure the width of the calcaneus. The hind leg central foot pad width may be measured using a digital caliper and laying the micrometer flat into the foot at the base of the pad. Hind leg central foot pad length may be measured using a digital caliper and laying the micrometer flat into the foot at the base of the pad. The front leg measurements are similar to the hind leg measurements, except the front legs are measured instead of the hind legs.

Head measurements may be provided as follows. The cranial length may be measured using a tailor's tape to measure from the exterior occipital protuberance to the medial canthus of the eye. The facial length may be measured using a tailor's tape to measure from the medial canthus of the eye to the tip of the nose. Head circumference may be measured using a tailor's tape to measure the circumference between the eyes and the ears at the widest part of the head. Finally, head width may be measured using the Seca measuring rod to measure between the eyes and ears.

After the measurements are recorded, multiple regression analysis may be applied using the DEXA results in order to develop regression equations for the prediction of lean body mass and fat mass from the measured body data and input descriptive data. The descriptive data can include anything from body weight, species, age, gender, neuter status, etc.

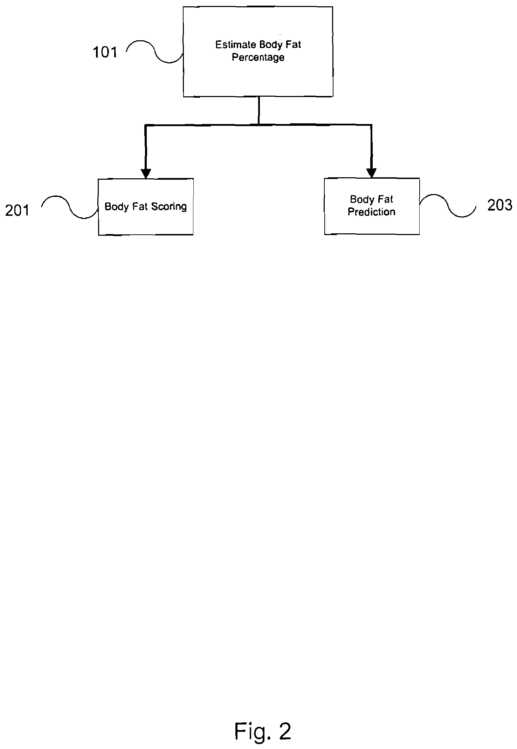

As described herein, two basic types of tools may be used to obtain an estimate of the body fat percentage of the animal. In accordance with **Fig. 2**, an exemplary method is provided called the Body Fat Scoring (BFS) method **201**. In the BFS method **201**, a visual and palpate assessment of body fat is made. This method uses the observations of a trained individual to assign a body fat index score to an individual animal. The body fat index score is generally understood to be a whole number that is an estimate of the percentage of body fat for that animal.

In one execution of the BFS method **201**, the animal is assessed using a chart that lists the characteristics for each body fat index category and is assigned a corresponding score. For instance, the body fat index score of 10 may indicate a range of 5-15% of body fat. The score of 10 requires that the ribs are prominent, easily felt, and contain little fat cover. The score of 10 also requires that the shape of the dog from above is a marked hourglass shape; the shape from the side is a pronounced abdominal tuck; the shape from behind is prominent bones and an angular contour; the tail base contains prominent bony structures, is easily felt, and contains little fat cover. The following table illustrates extensive categories of the body fat index score.

**Table 1: Body Fat Index**

| BFI Index | BFI Index | BFI Index | BFI Index | BFI Index | BFI Index |
|-----------|-----------|-----------|-----------|-----------|-----------|
| 10 | 20 | 30 | 40 | 50 | 60 |
| 5-15% BF | 15-25% BF | 25-35% BF | 35-45% BF | 45-55% BF | 55-65% BF |
|  |  |  |  |  |  |

(continued)

| Ribs | Ribs | Ribs | Ribs | Ribs | Ribs |
|---|---|---|---|---|---|
| Prominent; Easily felt; Little fat cover | Slightly prominent; easily felt; thin fat cover | Slightly to not prominent; can be felt; moderate fat cover | Not prominent; very difficult to feel; thick fat cover | Not prominent; extremely difficult to feel; very thick fat cover | Not prominent; impossible to feel; extremely thick fat cover |
| **Above Shape** | **Above Shape** | **Above Shape** | **Above Shape** | **Above Shape** | **Above Shape** |
| Marked hourglass shape | Well proportioned lumbar waist | Detectable lumbar waist | Loss of lumbar waist; broadened back | Markedly broadened back | Extremely broadened back |
| **Side Shape** | **Side Shape** | **Side Shape** | **Side Shape** | **Side Shape** | **Side Shape** |
| Pronounced abdominal tuck | Abdominal tuck present | Slight abdominal tuck | Flat to bulging abdomen | Marked abdominal bulge | Severe abdominal bulge |
| **Behind Shape** | **Behind Shape** | **Behind Shape** | **Behind Shape** | **Behind Shape** | **Behind Shape** |
| Prominent bones; angular contour | Clear muscle definition; smooth contour | Losing muscle definition; rounded appearance | Rounded to square appearance | Square appearance | Square appearance |
| **Tail Base** | **Tail Base** | **Tail Base** | **Tail Base** | **Tail Base** | **Tail Base** |
| Prominent bony structures; easily felt; little fat cover | Slightly prominent bony structures; easily felt; thin fat cover | Slightly to not prominent bony structures; can be felt; moderate fat cover | Bony structures are not prominent; very difficult to feel; thick fat cover | Bony structures are not prominent; extremely difficult to feel; very thick fat cover; fat dimple or fold present | Bony structures are not prominent; impossible to feel; extremely thick fat cover; large fat dimple or fat fold |

As expressed by the above table, each body fat index category covers a 10 point range in percentage of body fat. The body fat index score may then be entered into a weight loss calculator to obtain the ideal weight and feeding information.

As will be readily understood by a person having ordinary skill in the art, a way to describe this method is the subjective assessment of physical criteria based on multiple physical locations on the animal, with each assessment assigning a particular number of points. Once all the locations of the animal have been assessed, the points may be totaled to arrive at the estimated body fat index score. Then, the body fat index score may be entered into the weight loss calculator to obtain the ideal weight and feeding information.

The following table describes an exemplary body fat index scoring point system. When each of the criteria is evaluated by visual inspection and palpation, the total points may be combined.

Table 2: Body Fat Index Scoring Point System

| Criteria | | Description | | | | | | Point Assignment |
|---|---|---|---|---|---|---|---|---|
| | Points | 4 | 6 | 8 | 10 | 12 | 14 | |
| 1 | Ribs & Tail Base - Fat Cover | Thin | Minimal to moderate | Moderate to thick | Thick to very thick | Very thick to extremely thick | Extremely thick | |
| 2 | Ribs & Tail Base Palpation | Easily felt | Can be felt | Difficult to feel | Very difficult to feel | Extremely difficult to feel | Impossible to feel | |
| 3 | Shape from above | Well proportioned lumbar waist | Detectable lumbar waist | Loss of lumbar waist; broadened back | No lumbar waist; markedly broadened back | Lumbar bulge; markedly broadened back | Severe lumbar bulge; markedly broadened back | |
| 4 | Shape from the side | Abdominal tuck present | Abdominal tuck present | Slight to no abdominal tuck | Slight to moderate abdominal bulge | Severe abdominal bulge | Very severe abdominal bulge | |
| 5 | Shape from behind | Clear muscle definition; smooth contour | Losing muscle definition; rounded appearance | Rounded to square appearance | Square appearance; small to moderate fat dimple | Square appearance; large fat dimple | Square appearance; large fat fold at tail base | |
| | | | | | | | **Total Points (BFI)** | |

Improving the current BCS scale with the above BFS scale may provide for the correct food dose prescription for weight loss in severely obese companion animals. Moreover, a numerical point assignment methodology that allows the animal practitioner to enter data may be easily programmed into a Microsoft Excel spreadsheet, Microsoft Access database, or a similarly devised tool.

A second exemplary method for assessing the body fat percentage of the animal is the body fat prediction (BFP) method **203**. The BFP method **203** is the above described method that uses basic biological information and simple physical measurements to predict body fat and ideal body weight. This method can be described as formulating equations by using regression analysis techniques explained above, in order to predict the percentage of body fat or lean body mass based on physical data attainable by the practicing veterinarian. For instance, descriptive information such as body weight, age, gender, and neuter status may be combined with simple measurements (such as height, length, leg length, foot pad size, etc.) in order to arrive at an estimated body fat percentage.

According to an embodiment of the present invention, regression equations may be used to predict either lean body mass or fat mass. The percentage of body fat can then be calculated using either the lean body mass or the fat mass and the total body weight. The basic data required for body fat prediction may be entered into a BFP calculator which provides a tool for calculating the percentage of body fat and other body fat variables. The percentage of body fat can be entered into the same weight loss calculator as above or the weight loss calculations may be automatically incorporated into the BFP calculator. The BFP method **203** therefore provides an accurate and objective measurement, while maintaining a suitable format for the clinical setting.

The ideal body weight and food dose calculator may also be provided as a tool for calculating the RER and amount of food to daily feed the animal. For instance, the ideal weight calculator may receive as input the BFS score and the current body weight of the animal. Alternatively, the ideal weight calculator may receive as input the descriptive information. and equation parameters for the BFP method **203**. As an output, the ideal body weight calculator may determine the ideal weight of the animal, the RER calculation (i.e. kcal/day), and the amount of food to feed the animal. In addition, the ideal body weight and food dose calculator may determine the percentage of lean body mass and the amount of lean body mass, and alternatively display this information in spreadsheet format to the animal practitioner.

Alternatively, one may separate the spreadsheets for determining percent body fat and ideal body weight and determining the food dose based on the calculated information and the type of food. Likewise, separate spreadsheets may be used for any category of animal to which separate equations are to be applied. For instance, a table may be used to input morphometric measurements for dogs less than or equal to 18.144 kg (40 pounds), and a separate table may be used to input morphometric measurements for dogs greater than 18.144 kg (40 pounds). In this manner, separate equations may run the backend process whereby the output variables are calculated.

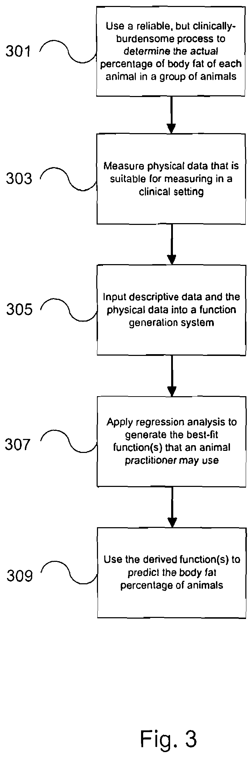

In Fig. 3, it is shown that for an exemplary process to be applied, one must first use a reliable, but clinically-burdensome process to determine the actual percentage of body fat of each animal in a group of animals **301**. Next, the user may measure physical data that is suitable for measuring in a clinical setting **303**. This allows the user to input the physically measured data, as well as descriptive data **305**, in order to derive a function suitable for the clinical setting. Regression analysis may then be used to generate the best-fit function(s) that the animal practitioner may use for the clinical setting **307**. Finally, the derived function(s) may be used to predict the body fat percentage of animals **309**.

Using a tool to predict the body fat percentage of an animal, the animal practitioner may then estimate ideal body weight, calculate the RER, and determine a daily food regimen for the animal in order to meet the ideal body weight goals.

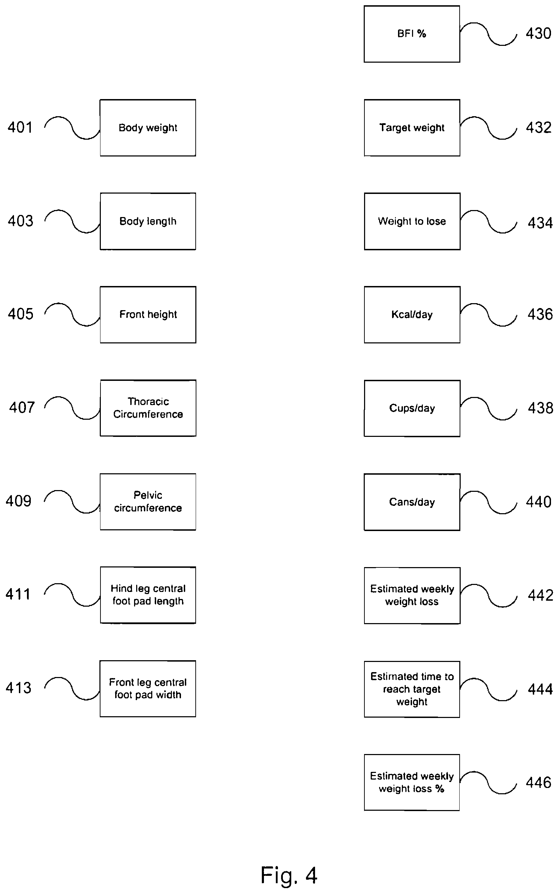

**Fig. 4** shows exemplary input and output parameters that may utilized in a preferred embodiment of a spreadsheet for dogs less than or equal to 18.144 kg (40 lbs). Body weight **401**, body length **403**, front height **405**, thoracic circumference **407**, pelvic circumference **409**, hind leg central foot pad length **411**, and front central foot pad width **413** are the parameters input into the spreadsheet in accordance with the above-described best fit algorithm for dogs less than or equal to 18.144 kg (40 lbs). Accordingly, the output parameters include BFI **430**, target weight **432**, weight to lose **434**, Kcal/day **436**, Cups/day **438**, Cans/day **440**, estimated weekly weight loss **442**, estimated time to reach target weight **444**, and the estimated weekly weight loss % **446**.

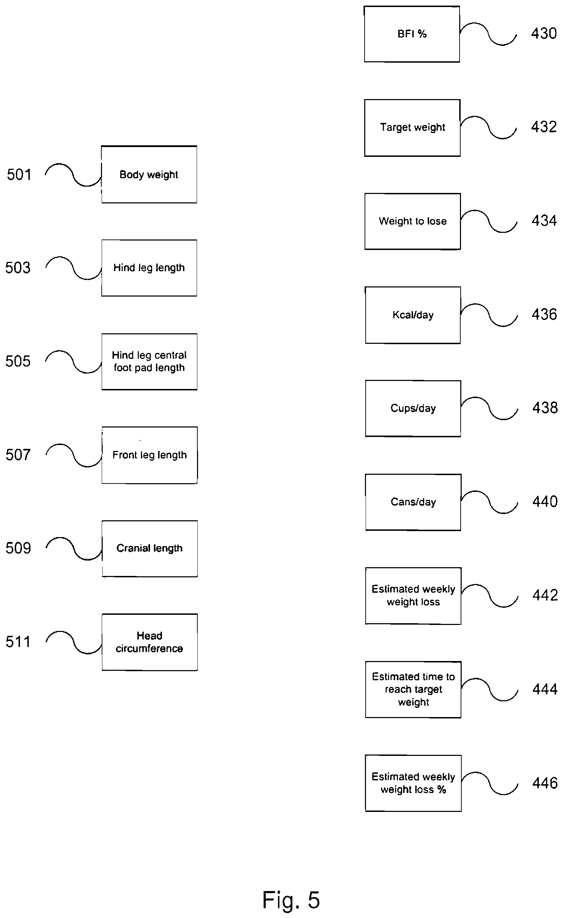

**Fig. 5** shows exemplary input and output parameters that may utilized in a preferred embodiment of a spreadsheet for dogs greater than 18.144 kg (40 lbs). Body weight **501**, hind leg length **503**, hind leg central foot pad length **505**, front leg length **507**, cranial length **509**, and head circumference **511** are the parameters input into the spreadsheet in accordance with the above-described best fit algorithm for dogs greater than 18.144 kg (40 lbs). Similarly, the output parameters include BFI **430**, target weight **432**, weight to lose **434**, Kcal/day **436**, Cups/day **438**, Cans/day **440**, estimated weekly weight loss **442**, estimated time to reach target weight **444**, and the estimated weekly weight loss % **446**.

Whether the BFS method or the BFP method is utilized to estimate the percentage of body fat of the animal, one should immediately realize improved dietary food prescriptions based on caloric intake, especially in overweight and obese animals.

## EXAMPLES

### Example 1

Thirty-six adult dogs with body composition ranging from overweight to morbidly obese were evaluated. The following measurements and procedures were conducted: body weight, palpation and visual assessment, digital photographs (front, rear, side and from above), body size and shape measurements, radiographs (head, thoracic and pelvic), and DEXA.

Lean body mass, fat mass and percent body fat were determined by DEXA. These data were used to evaluate other methods by providing the dependent variables to predict body composition (lean body mass, fat mass and percent body fat) by using independent variables obtained from morphometric measurements, skeletal measurements, body weight, age, gender, and neuter status. In this manner, equations to predict lean body mass, fat mass, and percent of fat were derived. Two separate models were applied. The first model was derived from the regression analysis using morphometric measurement. A second model was derived from the regression analysis using skeletal measurements.

### First Model: Morphometric measurements

Body size and shape (morphometric measurements) were used in regression analysis to predict body composition. The variables used in the analysis included body length, front height, rear height, thoracic circumference, pelvic circumference, front leg length, rear leg length, central foot pad length, central foot pad width, calcaneus width, head width, head circumference, facial length, and cranial length. Other variables included in the regression analysis were age, gender, and neuter status.

Stepwise multiple regression analysis was used to determine which morphometric variables provided the best estimate of lean body mass, fat mass, and percent body fat by DEXA. The data was analyzed with and without body weight as an independent variable. Models were developed for the entire study population and for two sub-populations, i.e., dogs with body weight less than or equal to 18.144 kg (40 pounds) and dogs with body weight greater than 18.144 kg (40 pounds).

With all dogs included in the regression analysis and weight included as an independent variable, the best model that was derived to predict lean body mass included the following parameters: body weight (BW), cranial length (CL), cranial length*head circumference (CL=x HC), head width (HW), hind leg center foot pad length (HLCFPL), calcaneus width (CW), hind leg length (HLL), pelvic circumference (PC), and front height (FH). In this equation, with the lean body mass being represented by LBM:

$$(1) \quad LBM = (134.4 \times BW) - (1012 \times CL) + (23.5 \times (CL \times HC)) - (403.7 \times HW) + (319.74 \times HLCFPL) - (214.8 \times CW) + (1012.4 \times HLL) - (30.34 \times PC) - (119.4 \times FH) + 2772.3.$$

Applying this model to the entire study population predicted lean body mass correctly in 83% of the dog population (within ± 10% of the DEXA value).

With all dogs included in the regression analysis and weight excluded as an independent variable, the best model that was derived to predict LBM included age, HLCFPL, PC, HC, front leg center foot pad width (FLCFPW), HLL, CL, and CL*HC. In this equation:

$$(2) \quad LBM = (122.5 \times age) + (174.33 \times HLCFPL) + (807.01 \times HLL) + (87.59 \times PC) - (570.1 \times HC) + (246.67 \times FLCFPW) - (2447 \times CL) + (58.92 \times (CL \times HC)) + 10712.$$

Applying this model to the entire study population predicted lean body mass correctly in 81% of the dog population (within ± 10% of the DEXA value).

For more accurate equations under the first model, the dogs were split into groups of those with body weight less than 18.144 kg (40 lbs.) and those with body weight greater than 18.144 kg (40 lbs). With all dogs having body weight less than 18.144 kg (40 lbs.) included in the regression analysis and weight included as an independent variable, the best model that was derived to predict LBM included age, BW, CL*HC, hind leg center food pad width (HLCFPW), CW, HLL and front leg length (FLL). In this equation:

$$(3) \quad LBM = (63.74 \times age) + (71.69 \times BW) + (5.31 \times (CL \times HC)) + (189.72 \times HLCFPW) - (122.8 \times CW) + (1019.6 \times HLL) - (337.7 \times FLL) - 4148.$$

Applying this model to the appropriate study population predicted lean body mass correctly in 100% of the respective dog population (within ± 10% of the DEXA value).

With all dogs having body weight less than 18.144 kg (40 lbs.) included in the regression analysis and weight excluded as an independent variable, the best model that was derived to predict LBM included age, body length (BL), CL*HC, HLL, FLL and facial length (FL). In this equation:

$$(4) \quad LBM = (60.22 \times age) + (111.3 \times BL) + (7.61 \times (CL \times HC)) + (1401.6 \times HLL) - (480.2 \times FLL) - (169 \times FL) - 5480.$$

Applying this model to the appropriate study population predicted lean body mass correctly in 100% of the respective dog population (within ± 10% of the DEXA value).

Similar techniques were applied to dogs with body weights greater than 18.144 kg (40 lbs). With all dogs having body weight greater than 18.144 kg (40 lbs.) included in the regression analysis and weight included as an independent variable, the best model that was derived to predict LBM included age, BW, CL*HC, CL, HLCFPL, HLL, and FLL. This equation is given by:

$$(5) \quad LBM = (-146.1 \times age) + (104.71 \times BW) + (15.31 \times (CL \times HC)) - (675 \times CL) + (394.04 \times HLCFPL) + (1239.4 \times HLL) - (372.4 \times FLL) - 6099.$$

Applying this model to the appropriate study population predicted lean body mass correctly in 100% of the respective dog population (within ± 10% of the DEXA value).

With all dogs having body weight greater than 18.144 kg (40 lbs.) included in the regression analysis and weight excluded as an independent variable, the best model that was derived to predict LBM included thoracic circumference (TC), PC, HLL, HLCFPL, FLL, and CL*HC. The equation is given by:

$$(6) \quad LBM = (148.92 \times TC) + (159.8 \times PC) + (944.01 \times HLL) + (679.12 \times HLCFPL) - (469.8 \times FLL) + (10.05 \times (CL \times HC)) - 31075.$$

Applying this model to the appropriate study population predicted lean body mass correctly in 95% of the respective dog population (within ± 10% of the DEXA value).

Fat mass may be calculated in a similar manner. With all dogs included in the regression analysis and weight included as an independent variable, the best model that was derived to predict fat mass (FM) included BW, CL*HC, HLCFPL, HLL, and TC. This equation is given by:

$$(7) \quad FM = (272.41 \times BW) - (7.54 \times (CL \times HC)) - (208.8 \times HLCFPL) - (463 \times HLL) + (98.25 \times TC) + 3110.3.$$

Applying the model to the entire study population predicted FM correctly in 78% of the dog population (within ± 10% of the DEXA value).

With all dogs included in the regression analysis and weight excluded as an independent variable, the best model that was derived to predict FM included TC, FLCFPL, and CW. This equation is given by:

$$(8) \quad FM = (366.14 \times TC) + (705.54 \times CW) - (365.1 \times FLCFPL) - 18496.$$

Applying this model to the entire study population predicted FM correctly in only 50% of the dog population (within ± 10% of the DEXA value).

Dividing the dogs into two separate groups based on body weight for the prediction of fat mass was also beneficial, similarly to predicting lean body mass. With all dogs having body weight less than 18.144 kg (40 lbs.) included in the regression analysis and with weight included as an independent variable, the best model derived to predict FM included BL, HLCFPL, FLCFPW, PC, TC, and front height (FH). This equation is given by:

$$(9) \quad FM = (185.29 \times BL) - (193.5 \times HLCFPL) - (49.75 \times FLCFPW) + (79.99 \times PC) +$$
$$162.51 \times TC - (49.72 \times FH) - 9129.$$

Applying this model to the appropriate study population predicted FM correctly in 100% of the respective dog population (within ± 10% of the DEXA value).

With all dogs having body weight less than 18.144 kg (40 lbs.) included in the regression analysis and weight excluded as an independent variable, equation (9) was found to be the best model and the predicted values were found to be the same.

With all dogs having body weights greater than 18.144 kg (40 lbs.) included in the regression analysis and weight included as an independent variable, the best model that was derived to predict FM included BW, HLL, HLCFPL, FLL, and CL*HC. This equation is given by:

$$(10) \quad FM = (303.25 \times BW) - (917.6 \times HLL) - (339.5 \times HLCFPL) + (298.28 \times FLL) - (6.68 \times (CL \times HQ) + 10067.$$

Applying this model to the appropriate study population predicted FM correctly in 100% of the respective dog population (within + 10% of the DEXA value).

Similarly, with all dogs having body weights greater than 18.144 kg (40 lbs.) included in the regression analysis and weight excluded as an independent variable, the best model that was derived to predict FM included TC, PC, HLL, and CW. This equation is given by:

$$(11) \quad FM = (343.17 \times TC) + (234.01 \times PC) - (566.6 \times HLL) + (465.59 \times CW) - 32291.$$

Applying this model to the appropriate study population predicted FM correctly in 64% of the respective dog population (within ± 10% of the DEXA value).

Percentage of fat may be calculated in a similar manner. With all dogs included in the regression analysis and weight included as an independent variable, the best model that was derived to predict percent fat (%Fat) included BL, RH, TC, HLL, CW, FLCFPW and HC. This equation is given by:

$$(12) \quad \%Fat = (0.44 \times BL) + (0.34 \times RH) + (0.81 \times TC) - (4.2 \times HLL) + (0.95 \times CW) -$$
$$(0.97 \times FLCFPL) - (1 \times HC) + 47.87.$$

Applying this model to the entire study population predicted %Fat correctly in 89% of the dog population (within ± 10% of the DEXA value).

With all dogs included in the regression analysis and weight excluded as an independent variable, equation (12) was found to be the best model and the predicted values were found to be the same.

Dividing the dogs into two separate groups based on body weight for the prediction of percentage fat was similarly beneficial. With all dogs having body weight less than 18.144 kg (40 lbs.) included in the regression analysis and with weight included as an independent variable, the best model derived to predict %Fat included age, PC, and HW. This equation is given by:

$$(13) \quad \%Fat = (1 \times PC) - (0.89 \times age) - (3.96 \times HW) + 35.81$$

Applying this model to the appropriate study population predicted %Fat correctly in 79% of the respective dog population (within + 10% of the DEXA value).

With all dogs having body weight less than 18.144 kg (40 lbs.) included in the regression analysis and with weight excluded as an independent variable, equation (13) was found to be the best model and the predicted values were found to be the same.

With all dogs having body weight greater than 18.144 kg (40 lbs.) included in the regression analysis and with weight included as an independent variable, the best model derived to predict %Fat included BW, FLL, CL*HC, HLCFPL, and HLL. This equation is given by:

$$(14)\ \%Fat = (0.24 \times BW) + (0.96 \times FLL) - (0.01 \times (CL \times HQ)) - (1.27 \times HLCFPL) - (2.62 \times HLL) + 79.55.$$

Applying this model to the appropriate study population predicted %Fat correctly in 100% of the respective dog population (within + 10% of the DEXA value).

With all dogs having body weight greater than 18.144 kg (40 lbs.) included in the regression analysis and with weight excluded as an independent variable, the best model derived to predict %Fat included PC and HLCFPL. This equation is given by:

$$(15)\quad \%Fat = (0.34 \times PC) - (1.12 \times HLCFPL) + 48.93.$$

Applying this model to the appropriate study population predicted %Fat correctly in 86% of the respective dog population (within $\pm$ 10% of the DEXA value).

**Second Model - Skeletal Measurement**

Radiographic data provided skeletal size information that was used in regression analysis to predict lean body mass. From the head, ventral-dorsal, and lateral radiographic views, the following were measured: facial length, cranial length, skull width, pelvic length, pelvic width, tibia length, tibia diameter, calcaneus length, and length from calcaneal tuber to distal end of metatarsal bones. In addition to these variables, the following variables were also included in the regression analysis: cranial length $\times$ head width, pelvic length $\times$ pelvic width, tibia length $\times$ tibia diameter, tibia area, tibia circumference, tibia volume, tibia surface area, and tibia total area.

With all dogs included in the regression analysis and weight included as an independent variable, the best model that was derived to predict lean body mass included the parameters cranial length (cranL), calcaneus length (calL), and body weight. This equation is given by:

$$(16)\quad LBM = (165.42 \times BW) + (2993.72 \times calL) - (442.01 \times cranL) - 4817.52.$$

Applying this model to the entire study population predicted lean body mass correctly in 72% of the dog population (within $\pm$ 10% of the DEXA value).

With all dogs included in the regression analysis and weight excluded as an independent variable, the best model that was derived to predict lean body mass included calL, head width (HW), and tibia area (TA). This equation is given by:

$$(17)\quad LBM = (3147.14 \times calL) + (1228.17 \times HW) + (24.39 \times TA) - 17171.7.$$

Applying this model to the entire study population predicted lean body mass correctly in only 47% of the dog population (within $\pm$ 10% of the DEXA value).

Dividing the dogs into two separate groups based on body weight for the prediction of lean body mass was similarly beneficial. With all dogs having body weight less than 18.144 kg (40 lbs.) included in the regression analysis and with weight included as an independent variable, the best model derived to predict lean body mass included cranL, HW, BW, cranL $\times$ HW, pelvic length $\times$ pelvic width (PL $\times$ PW), and tibia circumference (TC). This equation is given by:

$$(18)\quad LBM = (-3842.51 \times cranL) - (2737.71 \times HW) + (85.48 \times BW) + (422.51 \times (cranL \times HW)) + (16.33 \times (PL \times PW)) + (77.37 \times TC) + 23948.13.$$

Applying this model to the appropriate study population predicted lean body mass correctly in 100% of the respective dog population (within $\pm$ 10% of the DEXA value).

With all dogs having body weight less than 18.144 kg (40 lbs.) included in the regression analysis and with weight excluded as an independent variable, the best model derived to predict lean body mass included cranL × HW and calL. This equation is given by:

$$(19) \quad LBM = (50.38 \times (cranL \times HW)) + (2874.99 \times calL) - 7205.82.$$

Applying this model to the appropriate study population predicted lean body mass correctly in 57% of the respective dog population (within ± 10% of the DEXA value).

With all dogs having body weight greater than 18.144 kg (40 lbs.) included in the regression analysis and with weight included as an independent variable, the best model derived to predict lean body mass included cranL, calL, and BW. This equation is given by:

$$(20) \quad LBM = (-734.02 \times cranL) + (3460.67 \times calL) + (169.43 \times BW) - 4591.56.$$

Applying this model to the appropriate study population predicted lean body mass correctly in 86% of the respective dog population (within ± 10% of the DEXA value).

With all dogs having body weight greater than 18.144 kg (40 lbs.) included in the regression analysis and with weight excluded as an independent variable, the best model derived to predict lean body mass included HW and calL. This equation is given by:

$$(21) \quad LBM = (1513.35 \times HW) + (4790.33 \times calL) - 23102.8.$$

Applying this model to the appropriate study population predicted lean body mass correctly in 73% of the respective dog population (within ± 10% of the DEXA value).

Notably, in the above-described manner, the best equation for the prediction of lean body mass using skeletal size data and body weight resulted in an $r^2$ of 0.99 and a predictability (± 10%) of 100% for dogs less than or equal to 18.144 kg (40 lbs.) using 8 of the variables. These 8 variables were cranial length, head width, body weight, cranial length*head width, pelvic length*pelvic width, and tibia circumference. The best equation for the prediction of lean body mass using skeletal size data and body weight resulted in an $r^2$ of 0.99 and a predictability (± 10%) of 86% for dogs greater than 18.144 kg (40 lbs.) using 3 variables, namely cranial length, calcaneus length, and body weight.

Similarly, the best equation for prediction of lean body mass using body size data, body weight, and age resulted in an $r^2$ of 0.99 and a predictability (± 10%) of 100% for dogs less than or equal to 18.144 kg (40 lbs.) using 8 of the variables. These 8 variables included hind leg length, calcaneus width, hind leg central foot pad width, front leg length, cranial length*head circumference, body weight, and age. The best equation for prediction of lean body mass using body size data, body weight, and age resulted in an $r^2$ of 0.99 and a predictability (± 10%) of 100% for dogs greater than 18.144 kg (40 lbs.) using 7 of the variables, namely hind leg length, hind leg central foot pad length, front leg length, cranial length, cranial length*head circumference, body weight, and age.

Likewise, the best equation for prediction of fat mass resulted in an $r^2$ of 0.99 and a predictability (± 10%) of 100% for dogs less than or equal to 18.144 kg (40 lbs.) using body length, front height, thoracic circumference, pelvic circumference, hind leg central foot pad length, and front leg central foot pad width. The best equation for prediction of fat mass resulted in an $r^2$ of 0.97 and a predictability (± 10%) of 100% for dogs greater than 18.144 kg (40 lbs.) using hind leg length, hind leg central foot pad length, front leg length, cranial length*head circumference, and body weight.

The results of this study proved remarkable. First, it was determined that correlation existed between physically measurable attributes and the percent of body fat in already obese dogs. This allowed the study to conclude that multiple regression analysis may be applied to specific categories of animals in order to determine which clinically measurable attributes most strongly correlate to an accurate prediction of fat mass or lean body mass. In effect, this type of analysis gives the animal practitioner a practical yet effective tool for devising an accurate food regimen and healthy diet for the animal.

**Reference Example 1**

Lean body mass, fat mass and percent body fat were determined by DEXA. These data were used to evaluate other methods by providing the dependent variables to predict body weight, palpation and visual assessment, digital photographs (front, rear, side and from above), body size and shape measurements, radiographs (head, thoracic and pelvic) and DEXA.

Lean body mass, fat mass and percent body fat were determined by DEXA. This data was used to evaluate other methods by providing the dependent variables to predict body composition (lean body mass, fat mass and percent body fat) by using independent variables obtained from morphometric measurements, skeletal measurements, body weight, age, gender, and neuter status. In this manner, equations to predict lean body mass, fat mass, and percent of fat were derived. Two separate models were applied. The first model was derived from the regression analysis using morphometric measurement. A second model was derived from the regression analysis using skeletal measurements.

**First model: Morphometric measurements**

Body size and shape (morphometric measurements) were used in regression analysis to predict body composition. The variables used in the analysis included body length, front height, rear height, thoracic circumference, pelvic circumference, front leg length, rear leg length, central foot pad length, central foot pad width, calcaneus width, head width, head circumference, facial length, and cranial length. Other variables included in the regression analysis were age, gender, and neuter status.

Stepwise multiple regression analysis was used to determine which morphometric variables provided the best estimate of lean body mass, fat mass, and percent body fat by DEXA.

With all cats included in the regression analysis, the best model that was derived to predict lean body mass included the following parameters: head circumference (HC), front leg length (FLL), front leg circumference (FLC), and hind leg central food pad width (HLCFPW). In this equation, with the lean body mass being represented by LBM:

$$(22) \quad LBM = (-5270) + (147 \times HC) + (248 \times FLL) + (317 \times FLC) - (103 \times HLCFPW).$$

Fat mass may be calculated in a similar manner. With all cats included in the regression analysis and weight included as an independent variable, the best model that was derived to predict fat mass (FM) included body weight (BW), head circumference (HC), hind leg length (HLL), and front leg circumference (FLC). This equation is given by:

$$(23) \quad FM = (4910) + (438 \times BW) - (149 \times HC) - (296 \times HLL) - (206 \times FLC).$$

**Second Model - Skeletal Measurement**

Radiographic data provided skeletal size information that was used in regression analysis to predict lean body mass. From the head, ventral-dorsal, and lateral radiographic views, the following were measured: skull length, skull width, head length, head width, length from ileac crest to caudal edge of ischium, width from right to left ischitatic tuberosity, tibia length, tibia diameter, calcaneus length, and length from calcaneal tuber to distal end of metatarsal bones.

With all cats included in the regression analysis and gender included as an independent variable, the best model that was derived to predict lean body mass included the parameters: gender (G), head width (HW), pelvic length (PL), calcaneus length (calL), and calcaneal tuber length (calTL). This equation is given by:

$$(24) \quad LBM = -4630 + 301 \times G + 358 \times HW + 355 \times PL - 2240 \times calL + 871 \times calTL.$$

**Claims**

1. A method of predicting the percentage of lean body mass (LBM) in a companion animal, the method comprising: using measurements of physical data of the companion animal and applying one or more equations to predict the percentage of lean body mass in the companion animal, and **characterised in that** the measurements of physical data comprise measurements of body weight (BW), cranial length (CL), head circumference (HC), head width (HW), hind leg center foot pad length (HLCFPL), calcaneus width (CW), hind leg length (HLL), pelvic circumference (PC), and front height (FH),

   wherein the animal is a dog, and

   wherein the one or more equations used to predict lean body mass (LBM) include:

$$(1)\ LBM = (134.4 \times BW) - (1012 \times CL) + (23.5\ X\ (CL \times HC)) - (403.7 \times HW) + (319.74 \times$$
$$HLCFPL) - (214.8 \times CW) + (1012.4 \times HLL) - (30.34 \times PC) - (119.4 \times FH) + 2772.3.$$

2. The method of claim 1 wherein the one or more equations are two separate equations, wherein the first equation is used for a companion animal having a body weight equal to or less than a threshold amount, and the second equation is used for a companion animal having a body weight greater than a threshold amount.

3. The method of claim 2 wherein the threshold amount is 18.144 kg (forty pounds).

4. The method of claim 1, wherein the measurements of physical data further comprise measurements of age, head circumference (HC), front leg center foot pad width (FLCFPW), and,
   wherein the equations used to predict lean body mass (LBM) include:

$$(2)\ LBM = (122.5 \times age) + (174.33 \times HLCFPL) + (807.01 \times HLL) + (87.59 \times PC) - (570.1 \times$$
$$HC) + (246.67 \times FLCFPW) - (2447 \times CL) + (58.92 \times (CL \times HC)) + 10712.$$

5. The method of claim 3, wherein the dog has a body weight of less than 18.144 kg (40lbs) and,
   wherein the measurements of physical data further comprise measurements of age and front leg length (FLL), and wherein the equations used to predict lean body mass (LBM) include:

$$(3)\ LBM = (63.74 \times age) + (71.69 \times BW) + (5.31 \times (CL \times HC)) + (189.72 \times HLCFPW) -$$
$$(122.8 \times CW) + (1019.6 \times HLL) - (337.7 \times FLL) - 4148,$$

or,
wherein the measurements of physical data further comprise measurements of age, body length (BL), FLL and facial length (FL) and wherein the equations used to predict lean body mass (LBM) include:

$$(4)\ LBM = (60.22 \times age) + (111.3 \times BL) + (7.61 \times (CL \times HC)) + (1401.6 \times HLL) - (480.2 \times$$
$$FLL) - (169 \times FL) - 5480$$

or,
wherein the measurements of physical data further comprise measurements of pelvic length x pelvic width (PL x PW), and tibia circumference (TC), and wherein the equation used to predict lean body mass (LBM) include:

$$(18)\ LBM = (-3842.51 \times cranL) - (2737.71 \times HW) + (85.48 \times BW) + (422.51 \times (CL \times HW))$$
$$+ (16.33 \times (PL \times PW)) + (77.37 \times TC) + 23948.13$$

6. The method of claim 3, wherein the dog has a body weight of more than 18.144 kg (40lbs) and wherein the measurements of physical data further comprise measurements of age, and FLL and wherein the equations used to predict lean body mass (LBM) include:

$$(5)\ LBM = (-146.1 \times age) + (104.71 \times BW) + (15.31 \times (CL \times HC)) - (675 \times CL) + (394.04 \times$$
$$HLCFPL) + (1239.4 \times HLL) - (372.4 \times FLL) - 6099$$

or,
wherein the measurements of physical data comprise measurements of age, thoracic circumference (ThC), and FLL, and wherein the equations used to predict lean body mass (LBM) include:

$$(6)\ LBM = (148.92 \times ThC) + (159.8 \times PC) + (944.01 \times HLL) + (679.12 \times HLCFPL) - (469.8 \times FLL) + (10.05 \times (CL \times HC)) - 31075$$

or,
wherein the measurements of physical data further comprise measurements of calcaneus length (calL), and wherein the equations used to predict lean body mass (LBM) include:

$$(20)\ LBM = (-734.02 \times CL) + (3460.67 \times calL) + (169.43 \times BW) - 4591.56$$

7. The method of claim 1, further comprising applying one or more equations to predict the percentage of body fat (% fat) in the companion animal, wherein the measurements of physical data comprise measurements of BL, RH, TC, and FLCFPW, and the one or more equations used to determine body fat percentage (% fat) include:

$$(12)\ \%\ Fat = (0.44 \times BL) + (0.34 \times RH) + (0.81 \times TC) - (4.2 \times HLL) + (0.95 \times CW) - (0.97 \times FLCFPL) - (1 \times HC) + 47.87.$$

8. The method of claim 3, further comprising applying one or more equations to predict the percentage of body fat (% fat) in the companion animal, wherein the dog has a body weight of less than 18.144 kg (40lbs) and,
wherein the measurements of physical data further comprise measurements of age and the one or more equations used to determine body fat percentage (% fat) include:

$$(13)\ \%\ Fat = (1 \times PC) - (0.89 \times age) - (3.96 \times HW) + 35.81$$

9. The method of claim 3, further comprising applying one or more equations to predict the percentage of body fat (% fat) in the companion animal, wherein the dog has a body weight of more than 18.144 kg (40lbs) and,
wherein the measurements of physical data further comprise measurements of FLL, and the one or more equations used to determine body fat percentage (% fat) include:

$$(14)\ \%\ Fat = (0.24 \times BW) + (0.96 \times FLL) - (0.01 \times (CL \times HC)) - (1.27 \times HLCFPL) - (2.62 \times HLL) + 79.55,$$

or
wherein the one or more equations used to determine body fat percentage (% fat) include:

$$(15)\ \%\ Fat = (0.34 \times PC) - (1.12 \times HLCFPL) + 48.93$$

**Patentansprüche**

1. Ein Verfahren zum Vorhersagen des Prozentsatzes der Magermasse (LBM) bei einem Heimtier, wobei das Verfahren Folgendes beinhaltet: Verwenden von Messwerten körperlicher Daten des Heimtiers und Anwenden von einer oder mehreren Gleichungen, um den Prozentsatz der Magermasse bei dem Heimtier vorherzusagen, und **dadurch gekennzeichnet, dass** die Messwerte körperlicher Daten Messwerte des Körpergewichts (BW), Schädellänge (CL), Kopfumfang (HC), Kopfbreite (HW), Hinterbeinmitte-Fußballenlänge (HLCFPL), Fersenbeinbreite (CW), Hinterbeinlänge (HLL), Beckenumfang (PC) und Vorderhöhe (FH) beinhalten,
wobei das Tier ein Hund ist und
wobei die eine oder mehreren zum Vorhersagen der Magermasse (LBM) verwendeten Gleichungen die Folgenden umfassen:

$$(1)\ LBM = (134{,}4 \times BW) - (1012 \times CL) + (23{,}5 \times (CL \times HC)) - (403{,}7 \times HW) + (319{,}74 \times HLCFPL) - (214{,}8 \times CW) + (1012{,}4 \times HLL) - (30{,}34 \times PC) - (119{,}4 \times FH) + 2772{,}3.$$

2. Das Verfahren gemäß Anspruch 1, wobei die eine oder mehreren Gleichungen zwei separate Gleichungen sind, wobei die erste Gleichung für ein Heimtier mit einem Körpergewicht, das gleich einem Schwellenwert ist oder geringer als ein Schwellenwert ist, verwendet wird und die zweite Gleichung für ein Heimtier mit einem Körpergewicht, das höher als ein Schwellenwert ist, verwendet wird.

3. Das Verfahren gemäß Anspruch 2, wobei der Schwellenwert 18,144 kg (vierzig Pounds) beträgt.

4. Das Verfahren gemäß Anspruch 1, wobei die Messwerte körperlicher Daten ferner Messwerte des Alters, des Kopfumfangs (HC), der Vorderbeinmitte-Fußballenbreite (FLCFPW) beinhalten und wobei die zum Vorhersagen der Magermasse (LBM) verwendeten Gleichungen die Folgenden umfassen:

$$(2)\ LBM = (122{,}5 \times Alter) + (174{,}33 \times HLCFPL) + (807{,}01 \times HLL) + (87{,}59 \times PC) - (570{,}1 \times HC) + (246{,}67 \times FLCFPW) - (2447 \times CL) + (58{,}92 \times (CL \times HC)) + 10712.$$

5. Das Verfahren gemäß Anspruch 3, wobei der Hund ein Körpergewicht von weniger als 18,144 kg (40 lbs) aufweist und wobei die Messwerte körperlicher Daten ferner Messwerte des Alters und der Vorderbeinlänge (FLL) beinhalten und wobei die zum Vorhersagen der Magermasse (LBM) verwendeten Gleichungen die Folgenden umfassen:

$$(3)\ LBM = (63{,}74 \times Alter) + (71{,}69 \times BW) + (5{,}31 \times (CL \times HC)) + (189{,}72 \times HLCFPW)\ (122{,}8 \times CW) + (1019{,}6 \times HLL) - (337{,}7 \times FLL) - 4148$$

oder
wobei die Messwerte körperlicher Daten ferner Messwerte des Alters, der Körperlänge (BL), FLL und Gesichtslänge (FL) beinhalten und wobei die zum Vorhersagen der Magermasse (LBM) verwendeten Gleichungen die Folgenden umfassen:

$$(4)\ LBM = (60{,}22 \times Alter) + (111{,}3 \times BL) + (7{,}61 \times (CL \times HC)) + (1401{,}6 \times HLL) - (480{,}2 \times FLL) - (169 \times FL) - 5480$$

oder
wobei die Messwerte körperlicher Daten ferner Messwerte der Beckenlänge x Beckenbreite (PL x PW) sowie Tibiaumfang (TC) beinhalten und wobei die zum Vorhersagen der Magermasse (LBM) verwendeten Gleichungen die Folgenden umfassen:

$$(18)\ LBM = (-3842{,}51 \times cranL) - (2737{,}71 \times HW) + (85{,}48 \times BW) + (422{,}51 \times (CL \times HW)) + (16{,}33 \times (PL \times PW)) + (77{,}37 \times TC) + 23948{,}13$$

6. Das Verfahren gemäß Anspruch 3, wobei der Hund ein Körpergewicht von mehr als 18,144 kg (40 lbs) aufweist und wobei die Messwerte körperlicher Daten ferner Messwerte des Alters und FLL umfassen und wobei die zum Vorhersagen der Magermasse (LBM) verwendeten Gleichungen die Folgenden umfassen:

(5) $LBM = (-146,1 \text{ x Alter}) + (104,71 \text{ x BW}) + (15,31 \text{ x (CL x HC)}) - (675 \text{ x CL}) + (394,04 \text{ x HLCFPL}) + (1239,4 \text{ x HLL}) - (372,4 \text{ x FLL}) - 6099$

oder

wobei die Messwerte körperlicher Daten Messwerte des Alters, des Thoraxumfangs (ThC) und FLL beinhalten und wobei die zum Vorhersagen der Magermasse (LBM) verwendeten Gleichungen die Folgenden umfassen:

(6) $LBM = (148,92 \text{ x ThC}) + (159,8 \text{ x PC}) + (944,01 \text{ x HLL}) + (679,12 \text{ x HLCFPL}) - (469,8 \text{ x FLL}) + (10,05 \text{ x (CL x HC)}) - 31075$

oder

wobei die Messwerte körperlicher Daten ferner Messwerte der Fersenbeinlänge (calL) beinhalten und wobei die zum Vorhersagen der Magermasse (LBM) verwendeten Gleichungen die Folgenden umfassen:

(20) $LBM = (-734,02 \text{ x CL}) + (3460,67 \text{ x calL}) + (169,43 \text{ x BW}) - 4591,56$

**7.** Das Verfahren gemäß Anspruch 1, das ferner das Anwenden von einer oder mehreren Gleichungen beinhaltet, um den Körperfettanteil (% Fett) bei dem Heimtier vorherzusagen, wobei die Messwerte körperlicher Daten die Messwerte BL, RH, TC, und FLCFPW beinhalten und die eine oder mehreren zum Bestimmen des Körperfettanteils (% Fett) verwendeten Gleichungen die Folgenden umfassen:

(12) $\% \text{ Fett} = (0,44 \text{ x BL}) + (0,34 \text{ x RH}) + (0,81 \text{ x TC}) - (4,2 \text{ x HLL}) + (0,95 \text{ x CW}) - (0,97 \text{ x FLCFPL}) - (1 \text{ x HC}) + 47,87.$

**8.** Das Verfahren gemäß Anspruch 3, das ferner das Anwenden von einer oder mehreren Gleichungen beinhaltet, um den Körperfettanteil (% Fett) bei dem Heimtier vorherzusagen, wobei der Hund ein Körpergewicht von weniger als 18,144 kg (40 lbs) aufweist und

wobei die Messwerte körperlicher Daten ferner die Messwerte des Alters beinhalten und wobei die eine oder mehreren zum Bestimmen des Körperfettanteils (% Fett) verwendeten Gleichungen die Folgenden umfassen:

(13) $\% \text{ Fett} = (1 \text{ x PC}) - (0,89 \text{ x Alter}) - (3,6 \text{ x HW}) + 35,81$

**9.** Das Verfahren gemäß Anspruch 3, das ferner das Anwenden von einer oder mehreren Gleichungen beinhaltet, um den Körperfettanteil (% Fett) bei dem Heimtier vorherzusagen, wobei der Hund ein Körpergewicht von mehr als 18,144 kg (40 lbs) aufweist und

wobei die Messwerte körperlicher Daten ferner die Messwerte FLL beinhalten und wobei die eine oder mehreren zum Bestimmen des Körperfettanteils (% Fett) verwendeten Gleichungen die Folgenden umfassen:

(14) $\% \text{ Fett} = (0,24 \text{ x BW}) + (0,96 \text{ x FLL}) - (0,01 \text{ x (CL x HC)}) - (1,27 \text{ x HLCFPL}) - (2,62 \text{ x HLL}) + 79,55$

oder

wobei die eine oder mehreren zum Bestimmen des Körperfettanteils (% Fett) verwendeten Gleichungen die Folgenden umfassen:

$$(15) \ \% \ Fett = (0,34 \ x \ PC) - (1,12 \ x \ HLCFPL) + 48,93$$

**Revendications**

1. Méthode de prédiction du pourcentage de masse maigre de l'organisme (MMO) chez un animal de compagnie, la méthode comprenant : l'utilisation de mesures de données physiques de l'animal de compagnie et l'application d'une équation ou de plusieurs équations servant à prédire le pourcentage de masse maigre de l'organisme chez l'animal de compagnie, et **caractérisée en ce que** les mesures des données physiques comprennent les mesures du poids corporel (PC), la longueur crânienne (LCrân), le tour de tête (TT), la largeur de tête (LargT), la longueur du coussinet plantaire central de la patte arrière (LCPCPAr), la largeur du calcanéum (LargC), la longueur de la patte arrière (LPAr), le périmètre pelvien (PP) et la hauteur frontale (TF),
   dans laquelle l'animal est un chien, et
   dans laquelle l'équation ou les plusieurs équations utilisée(s) pour prédire la masse maigre de l'organisme (MMO) incluent :

$$(1) \ MMO = (134,4 \ x \ PC) - (1012 \ x \ LCrân) + (23,5 \ X \ (LCrân \ x \ TT) - (403,7 \ x \ LargT) + (319,74 \ x \ LCPCPA) - (214,8 \ x \ LargC) + (1012,4 \ x \ LPA) - (30,34 \ x \ PP) - (119,4 \ x \ HF) + 2772,3$$

2. Méthode selon la revendication 1, dans laquelle la ou les équation(s) sont deux équations séparées, dans laquelle la première équation est utilisée pour un animal de compagnie ayant un poids corporel inférieur ou égal à un poids seuil, et la seconde équation est utilisée pour un animal de compagnie ayant un poids corporel supérieur à un poids seuil.

3. Méthode selon la revendication 2, dans laquelle le poids seuil est de 18,144 kg (quarante livres).

4. Méthode selon la revendication 1, dans laquelle les mesures des données physiques comprennent, en outre, les mesures de l'âge, du tour de tête (TT), la largeur du coussinet plantaire central de la patte avant (LargCPCPAv), et dans laquelle les équations utilisées pour prédire la masse maigre de l'organisme (MMO) incluent :

$$(2) \ MMO = (122,5 \ x \ âge) + (174,33 \ x \ LCPCPAr) + (807,01 \ x \ LPAr) + (87,59 \ x \ PP) \ - (570,1 \ x \ TT) + (246,67 \ x \ LargCPCPAv) - (2447 \ x \ LCrân) + (58,92 \ x \ (LCrân \ x \ TT) + 10712.$$

5. Méthode selon la revendication 3, dans laquelle le chien a un poids corporel inférieur à 18, 144 kg (40 livres) et, dans laquelle les mesures des données physiques comprennent, en outre, les mesures de l'âge et la longueur de la patte avant (LPAv), et dans laquelle les équations utilisées pour prédire la masse maigre de l'organisme (MMO) incluent :

$$(3 \ ) \ MMO = (63,74 \ x \ âge) + (71,69 \ x \ PC) + (5,31 \ x \ (LCrân \ x \ TT) + (189,72 \ x \ LCPCPAr) - (122,8 \ x \ LarC) + (1019,6 \ x \ LPAr) - (337,7 \ x \ LPav) - 4148,$$

ou,
dans laquelle les mesures des données physiques comprennent, en outre, les mesures de l'âge, la longueur du corps (LC), la LPAv et la longueur faciale (LF), et dans laquelle les équations utilisées pour prédire la masse maigre de l'organisme (MMO) incluent :

(4 ) MMO = (60,22 x âge) + (111,3 x LC) + (7,61 x (Lcrân x TT)) + (1401,6 x LPAr) – (480,2 x LPAv) – (169 x LF) - 5480

ou
dans laquelle les mesures des données physiques comprennent, en outre, les mesures de la longueur pelvienne x largeur pelvienne (LP x LargP), et le périmètre du tibia (PT), et dans laquelle l'équation utilisée pour prédire la masse maigre de l'organisme (MMO) inclut :

(18) MMO = (-3842,51 x LCrân) – (2737,71 x LargT) + (85,48 x PC) + (422,51 x (LCrân x LargP)) + (16,33 x (LP x LargP)) + (77,37 x PT)  + 23948,13

6.   Méthode selon la revendication 3, dans laquelle le chien a un poids corporel supérieur à 18,144 kg (40 livres) et dans laquelle les mesures des données physiques comprennent, en outre, les mesures de l'âge, et la LPAv, et dans laquelle les équations utilisées pour prédire la masse maigre de l'organisme (MMO) inclut :

(5) MMO = (-146,1 x âge) + (104,71 x PC) + (15,31 x (LCrân x TT) – (675 x LCrân) + (397,04 x LCPCPAr) + (1239,4 x LPAr) – (372,4 x LPAv) – 6099

ou,
dans laquelle les mesures des données physiques comprennent les mesures de l'âge, le périmètre du thorax (PTh), et la LPav, et dans laquelle les équations utilisées pour prédire la masse maigre de l'organisme incluent :

(6) MMO = (148,92 x PTh) + (159,8 x PP) + (944,01 x LPAr) + (679,12 x LCPCPAr) – (469,8 x LPAv) + (10,05 x (LCrân x TT)) – 31075

ou,
dans laquelle les mesures des données physiques comprennent, en outre, les mesures de la longueur du calcanéum (LCal), et dans laquelle les équations utilisées pour prédire la masse maigre de l'organisme (MMO) incluent :

(20) MMO = (-734,02 x LCrân) + (3460,67 x LCal) + (169,43 x PC) – 4591,56

7.   Méthode selon la revendication 1 comprenant, en outre, l'application de l'une ou de plusieurs équations pour prédire le pourcentage de graisses corporelles (% de graisses) chez l'animal de compagnie, dans laquelle les mesures des données physiques comprennent les mesures de la LC, HA, le PT et la LargCPCPav, et l'équation ou les plusieurs équation(s) utilisée(s) pour déterminer le pourcentage de graisses corporelles (% de graisses) incluent :

(12) % de graisses = (0,44 x LC) + (0,34 x HA)  + (0,81 x PT) – (4,2 x LPAr) + (0,95 x LargC) -  (0,97 x LCPCPav) – (1 x TT) + 47,87.

8.   Méthode selon la revendication 3 comprenant, en outre, l'application de l'une ou de plusieurs équations pour prédire le pourcentage de graisses corporelles (% de graisses) chez l'animal de compagnie, dans laquelle le chien a un poids corporel inférieur à 18,144 kg (40 livres) et, sans laquelle les mesures des données physiques comprennent, en outre, les mesures de l'âge et l'équation ou les plusieurs d'équations utilisées pour déterminer le pourcentage de graisses corporelles (% de graisses) incluent :

(13) % de graisses = (1 x PP) – (0,89 x âge) – (3,96 x LargT) + 35,81

9. Méthode selon la revendication 3 comprenant, en outre, l'application de l'une ou de plusieurs équations pour prédire le pourcentage de graisses corporelles (% de graisses) chez l'animal de compagnie, dans laquelle le chien a un poids corporel supérieur à 18,144 kg (40 livres) et
dans laquelle les mesures des données physiques comprennent, en outre, les mesures de la LPAv, et l'équation ou les plusieurs équations utilisée(s) pour déterminer le pourcentage de graisses corporelles (% de graisses) incluent :

(14) % de graisses = (0,24 x PC) + (0,96 x LPAv) – (0,01 x (LCrân x TT)) – (1,27 x LCPCPar) – (2,62 x LPAr) + 79,55

ou
dans laquelle l'équation ou les plusieurs équations utilisée(s) pour déterminer le pourcentage de graisses corporelles (% de graisses) incluent :

(15) % de graisses = (0,34 x PP) – (1,12 x LCPCPAr) + 48,93

101 — Assess pet using tools to estimate body fat percentage

103 — Use body fat percentage to provide effective weight loss plan

105 — Determine ideal body weight, RER, daily feeding amount, and expected weight loss

Fig. 1

Fig. 2

301 — Use a reliable, but clinically-burdensome process to determine the actual percentage of body fat of each animal in a group of animals

303 — Measure physical data that is suitable for measuring in a clinical setting

305 — Input descriptive data and the physical data into a function generation system

307 — Apply regression analysis to generate the best-fit function(s) that an animal practitioner may use

309 — Use the derived function(s) to predict the body fat percentage of animals

Fig. 3

401 — Body weight

403 — Body length

405 — Front height

407 — Thoracic Circumference

409 — Pelvic circumference

411 — Hind leg central foot pad length

413 — Front leg central foot pad width

BFI % — 430

Target weight — 432

Weight to lose — 434

Kcal/day — 436

Cups/day — 438

Cans/day — 440

Estimated weekly weight loss — 442

Estimated time to reach target weight — 444

Estimated weekly weight loss % — 446

Fig. 4

501 — Body weight

503 — Hind leg length

505 — Hind leg central foot pad length

507 — Front leg length

509 — Cranial length

511 — Head circumference

BFI % — 430

Target weight — 432

Weight to lose — 434

Kcal/day — 436

Cups/day — 438

Cans/day — 440

Estimated weekly weight loss — 442

Estimated time to reach target weight — 444

Estimated weekly weight loss % — 446

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004068379 A
- WO 2005089567 A